# EUROPEAN PATENT APPLICATION

(11) **EP 4 124 659 A1**
(43) Date of publication of application: **01.02.2023**
(21) Application number: 21382710.8
(22) Date of filing: 29.07.2021
(51) Int. Cl.: C12N 15/115, A61K 31/7088, C12Q 1/70

(54) **APTAMERS AGAINST THE HEPATITIS C VIRUS CORE PROTEIN**

(71) Applicant: Consejo Superior De Investigaciones Científicas (CSIC), 28006 Madrid (ES); INTA, Instituto Nacional de Técnica Aeroespacial, 28850 Torrejon de Ardoz (ES); Aptus Biotech, S. L., 28035 Madrid (ES); Fundación para la Investigación Biomédica del Hospital Universitario Ramón y Cajal, 28034 Madrid (ES)
(72) Inventor: Briones Llorente, Carlos, 41013 Sevilla (ES); De Lucas Cerrillo, Ana María, 41013 Sevilla (ES); Fragoso Luna, Adrián, 41013 Sevilla (ES); Moreno Molina, Miguel, 41013 Sevilla (ES); Fernández Algar, María, 28850 Torrejón de Ardoz (Madrid) (ES); García Sacristán, Ana, 28035 Madrid (ES); González Muñoz, Víctor Manuel, 28034 Madrid (ES); Marín Palma, Elena, 28034 Madird (ES); Torres Vázquez, Beatriz, 28850 Torrejón de Ardoz (Madrid) (ES)
(74) Representative: Pons

(57) **Abstract**

The invention relates to aptamers which specifically bind to core protein of the hepatitis C virus, particularly to the region between amino acids 1 and 122 of the core protein, and their uses for detecting or diagnosis hepatitis C, as well as for using said aptamers as a medicament.

## Description

The invention relates to aptamers which specifically bind to the core protein of the hepatitis C virus (HCV), particularly to the region between amino acids 1 and 122 of the core protein, and their uses for detecting or diagnosis hepatitis C, as well as for using said aptamers as a medicament.

### BACKGROUND ART

HCV affects about 115 million people on five continents (2-3% of the worldwide population) and 350,000 infected individuals die every year due to complications derived from hepatitis C, including cirrhosis and hepatocellular carcinoma.

Currently, the diagnosis of HCV infection is performed by means of systems based on the detection of the anti-HCV antibodies produced by the infected host (immunologic tests), or of molecular components (antigens) of the virus such as proteins or viral genomic RNA (antigenic tests). In the first case, there are different commercial ELISA (Enzyme-Linked ImmunoSorbent Assay) kits which can be used as a method of sieving. Nevertheless, they can give false negatives, thus requiring confirmation by means of another complementary technique, which is generally based on the detection of the viral genomic RNA using variants of the RT-PCR.

Regarding the treatment options for the HCV, the recent inclusion of direct acting antivirals (DAAs) in the pharmacological repertory has clearly improved the response of the patients, drastically reducing the morbimortality associated with the infection by this virus. Nevertheless, these drugs are already generating resistance mutations, they can cause side effects, and, furthermore, the high price thereof makes them an option largely inviable in countries with a higher prevalence of infection by HCV.

Aptamers are oligomers of single-stranded nucleic acids (ssDNA or RNA) which are obtained from combinatorial libraries by means of *in vitro* evolution, the three-dimensional structure of which enables them to bind with high affinity and specificity to the desired target molecule. For example, the patent application CN102121009 A discloses a ssDNA aptamer that binds to HCV core protein. By using such an aptamer, HCV core protein can be captured and detected in solution, and hepatitis C serodiagnosis and blood screening can be performed.

Alternatively, owing to the advantages of straightforward preparation, easy chemical modification and good stability, the aptamers can be used as novel biorecognition probes for biosensors and diagnosis systems which are quick, cheap and eventually more sensitive than the current ones (mainly based on antibodies). Aptamers have been used to construct biosensors for the detection of pathogenic microorganisms (protozoa, viruses, bacteria) (Wang Yi-Xian et al., Chinese Journal of Analytical Chemistry, 2012; 40(4): 634-642; Ling Sum Liu et al. ACS Appl. Mater. Interfaces 2021, 13, 9329-9358).

Lee S., et al. (Biochemical and Biophysical Research Communications 2007 358:47-52) disclose the selection of RNA aptamers that bind to the core antigen of HCV. The selected aptamers specifically bind to the core antigen, but not to another HCV antigen, NS5, in a protein chip-based assay. Using these aptamers, an aptamer-based biosensor for HCV diagnosis and detection in infected patients' sera has been developed.

In other example, Pleshakova T.O., et al. (Micromachines 2019; 10(2):129) disclose the detection of HCV in serum using aptamer-functionalized AFM chips. The target particles, containing HCV core (HCVcoreAg protein) were biospecifically captured onto the chip surface from test solution containing serum collected from a hepatitis C patient.

However, the aptamers against HCV disclosed in the state of the art have affinity values (measured as the value of the dissociation constant, Kd) around 100nM. Therefore, to improve HCV diagnosis and treatment it is necessary to select *in vitro* and optimize aptamers with higher sensitivity and affinity for HCV core, i.e., showing affinity values less than 100nM.

### DESCRIPTION OF THE INVENTION

The present invention discloses aptamers with high affinity for the hepatitis C virus (HCV) core protein, which forms the viral capsid. It is interesting to highlight that this is the least variable of all the coded proteins in the HCV genome, and that the aptamers specific bind to HCV core.

In the *in vitro* selection processes performed, six variants of the HCV core protein belonging to the four main genotypes of the virus (HCV-1, HCV-2, HCV-3 and HCV-4) were used. Two of them contained the full-length sequence of HCV core (amino acids 1 to 191), belonging to genotype 1: a C-terminal biotin-tagged, fusion protein with β-galactosidase (termed G1-191-F), and an N-terminal 6xHis-tagged protein expressed in a baculovirus-insect systems (termed G1-191). Additionally, the N-terminal 6xHis-tagged hydrophilic domain (amino acids 1 to 122) of HCV core expressed in a baculovirus-insect system and belonging to the four genotypes (termed G1-122, G2-122, G3-122 and G4-122, respectively) were used as targets for *in vitro* selection.

The characterisation of the affinity and specificity of the aptamers by the targets thereof was performed using different techniques, among them those based on ELONA (a system analogous to ELISA but using aptamers instead of antibodies) coupled to colorimetric detection (for ssDNA aptamers) or to amplification by real time, quantitative RT-PCR (for RNA aptamers).

The inventors performed comparative analyses of the results obtained enabling the identification of the sequences of 11 individual aptamers, between 74 and 77 nucleotides long, that showed higher affinity by the region spanning amino acids 1 to 122 (SEQ ID NO: 41) of the HCV core protein (SEQ ID NO: 42), with Kd values comprised between 0.4 and 197.2 nM (Table 1). These aptamers thus permit the detection of the HCV core protein in low nanomolar range.

The sequences of these aptamers share common flanking regions (hereinafter, the underlined sequence regions), around 18 nucleotides at the 5'-end and 18 nucleotides at the 3'-end, with a variable central zone of 38 to 41 nucleotides in length. Particularly, the group of aptamers termed Apt-HCVcore_01 to Apt-HCVcore_08 comprise the same flanking regions, while the aptamers Apt-HCVcore_09 to Apt-HCVcore_11 also contain common flanking regions (the underlined sequence areas), which present some differences with respect to those shared by Apt-HCVcore_01 to Apt-HCVcore_08 (Table 1). All these aptamers comprise a nucleotide consensus sequence SEQ ID NO: 1 (with a length of 74 nucleotides).

A first aspect of the present invention relates to an aptamer which specifically binds to the region consisting of amino acids between 1 and 122 of the core protein of HCV (SEQ ID NO: 41), wherein the aptamer comprises a nucleotide sequence with at least 80% identity with SEQ ID NO: 1, hereinafter the "aptamer of the invention":
SEQ ID NO:1 
GCGGATSMAGACTGGTGTDBNNNNNNBNBNNNNHBNNNDNBHNNHNBHNHY
BNDBGCCCTAAAKACRAGCWWC
 wherein letter "N" is any nucleotide; "S" is a nucleotide selected from C or G; "M" is a nucleotide selected from A or C; "K" is a nucleotide selected from G or T; "R" is a nucleotide selected from A or G; "B" is a nucleotide selected from G, C or T; "H" is a nucleotide selected from C, T or A; "D" is a nucleotide selected from G, T or A; "Y" is a nucleotide selected from C or T and "W" is a nucleotide selected from T or A.

The terms "nucleotide sequence", "nucleic acid", "isolated nucleotide sequence", "polynucleotide sequence" or "polynucleotide" are interchangeably used herein and refer to a nucleic acid molecule (either DNA or RNA, containing deoxyribonucleotides or ribonucleotides respectively), or to an artificial nucleic acid analogue [of the group composed of, but not limited to, glycerol-derived nucleic acid (GNA), threose nucleic acid (TNA), pyranosyl-RNA (p-RNA), locked nucleic acid (LNA), and peptide nucleic acid (PNA)], or to any combination of nucleic acids and said analogue molecules. The nucleic acid may be double stranded, single stranded, or contain portions of both double stranded or single stranded sequence.

Nucleotide sequence or polynucleotide sequence are broader terms than the term aptamer and, thus, the terms nucleotide sequence or polynucleotide sequence include aptamers but are not limited to aptamers.

As used herein, "aptamer" refers to a non-naturally occurring nucleic acid that has a desirable action on a target molecule. A desirable action includes, but is not limited to, non-covalently binding to the target, covalently attaching to the target, reacting with the target in a way that either modifies or alters the target or the functional activity of the target, and facilitating the reaction between the target and another molecule. The preferred action is specific binding to a target molecule, particularly, core protein of HCV.

In this context, the "specific binding affinity" of an aptamer for its target (such as HCV core protein) means that the aptamer binds to its target generally with a much higher affinity (i.e., much lower value of its Kd) than it binds to other, non-target, components in a mixture or sample. Preferably, the specific binding refers to a non-covalent physical association between two molecules, the aptamer of the invention and the core protein of HCV. The binding between the aptamer of the invention and the core protein of HCV is considered specific if the binding strength value between both is at least 10 times, at least 15 times, at least 20 times, at least 25 times, at least 50 times, at least 75 times or at least 100 times greater than the binding strength value (binding strength reference value or binding strength standard value) between the aptamer of the invention and an irrelevant molecule or a reference molecule.

As a person skilled in the art knows, the "irrelevant molecule" or "reference molecule" refers to any molecule not related (from the points of view of its sequence, structure or function) to HCV core protein wherein said molecule has non-specific affinity for the aptamer of the invention. Examples of irrelevant molecules or reference molecules include, without limitation to, bovine serum albumin, human albumin, collagen, actin, myosin, histones, ribosomal proteins, DNA molecules, RNA molecules, cholesterol, phospholipids, glucose and glycogen.

So, in a preferred embodiment the binding strength value between the aptamer of the invention and the core protein of HCV is at least 10 times, at least 15 times, at least 20 times, at least 25 times, at least 50 times, at least 75 times or at least 100 times greater than a reference value, wherein the reference value is the binding strength value between the aptamer of the invention and an irrelevant molecule.

The binding between the aptamer of the invention and the core protein of HCV is also considered specific if the equilibrium dissociation constant Kd is 10⁻³ M or less, 10⁻⁴ M or less, 10⁻⁵ M or less, 10⁻⁶ M or less, 10⁻⁷ M or less, 10⁻⁸ M or less, 10⁻⁹ M or less, 10⁻¹⁰ M or less, 10⁻¹¹ M or less, or 10⁻¹² M or less, under the conditions used for the binding. In a preferred embodiment, the aptamer of the invention binds to its target protein with a Kd of between 10⁻¹¹M and 10⁻⁸M.

The capability of the aptamer of the invention to specifically bind to the core protein of HCV can be determined by means of a range of assays that are available in the art. Preferably, the capability of the aptamer of the invention for the specific binding to the core protein of HCV is determined by means of *in vitro* binding assays, such as the enzyme-linked oligonucleotide assay (ELONA), the enzyme-linked aptamer sorbent assay (ELASA), quantitative PCR (qPCR), or by fluorescence techniques (such as aptahistochemistry, aptacytochemistry, fluorescence microscopy or flow cytometry). Likewise, both the capability of specific binding to the core protein of HCV and the affinity of the aptamer for such core protein can be determined by techniques well-known by the person skilled in the art, such as gel mobility shift assay, surface plasmon resonance (SPR), kinetic capillary electrophoresis and fluorescence binding assay. Briefly, the fluorescence binding assay consists of the incubation of magnetic balls coated with the core protein with different concentrations (for example, from 0 to 100 nM) of the aptamer of the invention labeled (for example, with carboxyfluorescein, FAM), and the subsequent elution and detection of the bound aptamers; the dissociation constants (Kd) are calculated by non-linear fit analysis.

The term "hepatitis C virus" or its acronym "HCV" as used herein refers to a small (55-65 nm in size), enveloped, positive-sense single-stranded RNA virus of the family *Flaviviridae.* HCV is the cause of hepatitis C, cirrhosis and some cancers such as liver cancer (hepatocellular carcinoma, abbreviated HCC) and lymphomas in humans. The HCV particle consists of a lipid membrane envelope, inside which is an icosahedral capsid that is 33 to 40 nm in diameter, inside which is the genomic RNA material of the virus. The icosahedral capsid is formed entirely by the HCV core protein, which is the target of the aptamer of the invention.

The term "hepatitis C virus core protein" or "HCV core protein" as used herein, refers to the protein that forms the capsid of the virus. As with most virus, the HCV core protein is the first part of a large polyprotein which results of the translation of the RNA genome of the virus. The genome polyprotein is then cleaved into the several components required for the RNA replication, virion formation, packaging of the virus and exit from the infected hepatocyte. The HCV core protein is first cleaved into the HCV core precursor protein, which corresponds to the amino acids 2 to 191 of the polyprotein. After this the HCV core protein matures into the final polypeptide which corresponds to the amino acids 2 to 177 of the polyprotein. The genetic variability of HCV has given rise to various virus genotypes, each of which contain closely related sequences. Among the common and widespread six genotypes of HCV, genotype 1 is the most prevalent (covering around 70-90% of total infections) while genotype 2 and genotype 3 are less common contributing to around 10-20% of total infections worldwide. Particularly, the aptamers of the present invention recognize the core protein of HCV genotypes 1 to 4 (GenBank accession numbers respectively: AFS60333.1 (30 September 2012) (SEQ ID NO: 42), AFS60360.1 (30 September 2012) (SEQ ID NO: 43), ACZ60107.1 (24 July 2016) (SEQ ID NO:44) and DQ988076.1 (14 July 2016) (SEQ ID NO: 45)).

The aptamers of the present invention recognize the region of the N-terminal end between amino acids 1 and 122 of the HCV core protein that belongs to genotype 1, which consists on the amino acid sequence SEQ ID NO: 41.

The aptamer of the invention comprises a nucleotide sequence with at least 80% identity with SEQ ID NO: 1. The terms "% sequence identity" or "% identity" refer to the percentage of nucleotides or amino acids of a candidate sequence that are identical to the nucleotides or amino acids in the sequence of reference, after aligning the sequences to achieve the maximum % sequence identity. The % sequence identity can be determined by any methods or algorithms established in the art, such as the ALIGN, BLAST and BLAST 2.0 algorithms. See (Altschul S, et al., Nuc Acids Res. 1977; 25:3389-3402), (Altschul S, et al., J Mol Biol. 1990;215:403-410) and (McGinnis S. and Madden T.L., Nucleic Acids Res. 2004; 32: W20-W25). In certain embodiments, the percentage identity "X" of a first nucleic acid sequence with respect to a second nucleic acid sequence is calculated as 100 x (Y/Z), where Y is the number of nucleic acids residues scored as identical matches in the alignment of the first and second sequences (as aligned by visual inspection or a particular sequence alignment program) and Z is the total number of nucleic acid residues in the second sequence. If the second sequence is longer than the first sequence, then the percent identity may be determined only in the region of overlap between said first and second sequences. In this case, the same formula as above can be applied but using as Z value the length of the region wherein the first and second sequence overlap, said region having a length which is substantially the same as the length of the first sequence.

In a preferred embodiment, the aptamer of the invention comprises a nucleotide sequence with at least 85%, at least 90%, at least 91%, at least 92%, at least 93%, at least 94%, at least 95%, at least 96%, at least 97%, at least 98%o or at least 99% identity with SEQ ID NO: 1. In other preferred embodiment, the aptamer of the invention comprises or consists on the nucleotide sequence SEQ ID NO: 1.

The length of the aptamer of the present invention is not particularly limited, and can typically be between 10 to 150 nucleotides. In other preferred embodiment of the invention the length of the aptamer of the invention is between 50 to 100 nucleotides, preferably between 60 to 90, more preferably between 65 to 85 nucleotides.

In other more preferred embodiment of the invention the length of the aptamer of the invention is 66, 67, 68, 69, 70, 71, 72, 73, 74, 75, 76, 77, 78, 79, 80, 81, 82, 83 or 84 nucleotides, preferably 74, 75, 76 or 77 nucleotides. Preferably, in other preferred embodiment of the invention, the length of the aptamer comprises or consists of the nucleotide sequence SEQ ID NO: 1 is 74 nucleotides.

As a person skilled in the art knows, the aptamer of the invention may be the complementary reverse sequence SEQ ID NO: 1. The terms "complementary reverse sequence" or "reverse complement sequence" used herein refers to the nucleotide sequence that is formed by assembling all of the complementary nucleotides corresponding to the aptamer of the invention, positioned in the reverse orientation with respect to the meaning sequence.

Thus, in other preferred embodiment of the invention, the aptamer of the invention comprises a nucleotide sequence with at least 80% identity with SEQ ID NO: 21. In other preferred embodiment, the aptamer of the invention comprises a nucleotide sequence with at least 85%, at least 90%, at least 91%, at least 92%, at least 93%, at least 94%, at least 95%, at least 96%, at least 97%, at least 98%o or at least 99% identity with SEQ ID NO: 21.

In other preferred embodiment, the aptamer of the invention comprises or consists on the nucleotide sequence SEQ ID NO: 21.
SEQ ID NO: 21 
GWWGCTYGTMTTTAGGGCVHNVRDNDVNDNNDVNHNNNNVDNNNNVNVNNNN
NNVHACACCAGTCTKSATCCGC
 wherein letter "N" is any nucleotide; "S" is a nucleotide selected from C or G; "M" is a nucleotide selected from A or C; "K" is a nucleotide selected from G or T; "R" is a nucleotide selected from A or G; "H" is a nucleotide selected from C, T or A; "D" is a nucleotide selected from G, T or A; "Y" is a nucleotide selected from C or T; "V" is a nucleotide selected from G, C or A and "W" is a nucleotide selected from T or A.

The variable central region of the aptamer of the invention (SEQ ID NO: 1 or SEQ ID NO: 21) may comprise various more nucleotides, thus producing a nucleotide consensus sequence with 75 nucleotides in length (SEQ ID NO: 2) or with 76 nucleotides in length (SEQ ID NO: 3). Further, the common flanking regions of the aptamer of the invention (the underlined sequence areas) may comprise one more nucleotide at the 3'-end, producing a nucleotide consensus sequence 77 nucleotides in length (SEQ ID NO: 4).

Thus, in other preferred embodiment of the invention, the aptamer comprises or consists of the nucleotide sequence SEQ ID NO: 2, SEQ ID NO: 3 or SEQ ID NO: 4.
SEQ ID NO: 2 
GCGGATSMAGACTGGTGTDBNNNNNNBNBNNNNHBNNNNDNBHNNHNBHNHY
BNDBBGCCCTAAAKACRAGCWWC
SEQ ID NO: 3 
GCGGATSMAGACTGGTGTDBNNNNNNBNBNNNNHBNNNNDNBHNNHNBHNHY
BNDBBBGCCCTAAAKACRAGCWWC
SEQ ID NO: 4 
GCGGATSMAGACTGGTGTDBNNNNNNBNBNNNNHBNNNDNBHNNHNBHNHY
BNDBBBGCCCTAAAKAGCRAGCWWC
wherein letter "N" is any nucleotide; "S" is a nucleotide selected from C or G; "M" is a nucleotide selected from A or C; "K" is a nucleotide selected from G or T; "R" is a nucleotide selected from A or G; "B" is a nucleotide selected from G, C or T; "H" is a nucleotide selected from C, T or A; "D" is a nucleotide selected from G, T or A; "Y" is a nucleotide selected from C or T and "W" is a nucleotide selected from T or A.

As described above, the aptamer of the invention may be the complementary reverse sequence of nucleotide sequences SEQ ID NO: 2, SEQ ID NO: 3 or SEQ ID NO: 4.

So, in other preferred embodiment of the invention, the aptamer comprises or consists on the nucleotide sequence SEQ ID NO: 22, SEQ ID NO: 23 or SEQ ID NO: 24.
SEQ ID NO: 22 
GWWGCTYGTMTTTAGGGCVVHNVRDNDVNDNNDVNHNNNNVDNNNNVNVNNN
NNNVH ACACCAGTCTKSATCCGC
SEQ ID NO: 23 
GWWGCTYGTMTTTAGGGCVVVHNVRDNDVNDNNDVNHNNNNVDNNNNVNVNN
NNNNVHACACCAGTCTKSATCCGC
SEQ ID NO: 24 
GWWGCTYGCTMTTTAGGGCVVVHNVRDNDVNDNNDVNHNNNNVDNNNNVNVN
NNNNNVHACACCAGTCTKSATCCGC
wherein letter "N" is any nucleotide; "S" is a nucleotide selected from C or G; "M" is a nucleotide selected from A or C; "K" is a nucleotide selected from G or T; "R" is a nucleotide selected from A or G; "H" is a nucleotide selected from C, T or A; "D" is a nucleotide selected from G, T or A; "Y" is a nucleotide selected from C or T; "V" is a nucleotide selected from G, C or A and "W" is a nucleotide selected from T or A.

Particularly, the aptamers Apt-HCVcore_01 to Apt-HCVcore_08 (Table 1) share common flanking regions (the underlined sequence areas), wherein the 5'-end comprises the nucleotide sequence GCGGATCCAGACTGGTGT (SEQ ID NO: 46) and the 3'-end comprises the nucleotide sequence GCCCTAAAGACAAGCTTC (SEQ ID NO: 47), obtaining a nucleotide consensus sequence of the aptamers Apt-HCVcore_01 to Apt-HCVcore_08, the nucleotide consensus sequence SEQ ID NO: 5.

So, in other preferred embodiment of the invention, the aptamer comprises or consists on the nucleotide sequence SEQ ID NO: 5
SEQ ID NO: 5 
GCGGATCCAGACTGGTGTRBNNNNNNBNBNNNBHBNNNNDNBHNNHNBHNHY
BNWBGCCCTAAAGACAAGCTTC
 wherein letter "N" is any nucleotide; "R" is a nucleotide selected from A or G; "B" is a nucleotide selected from G, C or T; "H" is a nucleotide selected from C, T or A; "D" is a nucleotide selected from G, T or A; "Y" is a nucleotide selected from C or T and "W" is a nucleotide selected from T or A.

As described above, the aptamer of the invention may be the complementary reverse sequence of nucleotide sequences SEQ ID NO: 5.

So, in other preferred embodiment of the invention, the aptamer comprises or consists on the nucleotide sequence SEQ ID NO: 25.
SEQ ID NO: 25 
GAAGCTTGTCTTTAGGGCVWNVRDNDVNDNNDVNHNNNNVDVNNNVNVNNNN
NNVYACACCAGTCTGGATCCGC
 wherein letter "N" is any nucleotide; "R" is a nucleotide selected from A or G; "H" is a nucleotide selected from C, T or A; "D" is a nucleotide selected from G, T or A; "Y" is a nucleotide selected from C or T; "V" is a nucleotide selected from G, C or A and "W" is a nucleotide selected from T or A.

The variable central region of the aptamer of the invention, particularly of the aptamer with SEQ ID NO: 5, may comprise various more nucleotide obtaining a nucleotide consensus sequence with 75 nucleotides length (SEQ ID NO: 6) or with 76 nucleotides length (SEQ ID NO: 7).

So, in other more preferred embodiment of the invention, the aptamer comprises or consists on the nucleotide sequence SEQ ID NO: 6 or SEQ ID NO: 7.
SEQ ID NO: 6 
GCGGATCCAGACTGGTGTRBNNNNNNBNBNNNBHBNNNNDNBHNNHNBHNHY
BNWBBGCCCTAAAGACAAGCTTC
SEQ ID NO: 7 
GCGGATCCAGACTGGTGTRBNNNNNNBNBNNNBHBNNNNDNBHNNHNBHNHY
BNWBBBGCCCTAAAGACAAGCTTC
wherein letter "N" is any nucleotide; "R" is a nucleotide selected from A or G; "H" is a nucleotide selected from C, T or A; "D" is a nucleotide selected from G, T or A; "Y" is a nucleotide selected from C or T; "V" is a nucleotide selected from G, C or A and "W" is a nucleotide selected from T or A.

As described above, the aptamer of the invention may be the complementary reverse sequence of nucleotide sequences SEQ ID NO: 6 or SEQ ID NO: 7. So, in other preferred embodiment of the invention, the aptamer comprises or consists on the nucleotide sequence SEQ ID NO: 26 or SEQ ID NO: 27.
SEQ ID NO: 26 
GAAGCTTGTCTTTAGGGCVVWNVRDNDVNDNNDVNHNNNNVDVNNNVNVNNN
NNNVY ACACCAGTCTGGATCCGC
SEQ ID NO: 27 
GAAGCTTGTCTTTAGGGCVVVWNVRDNDVNDNNDVNHNNNNVDVNNNVNVNN
NNNNVYACACCAGTCTGGATCCGC
wherein letter "N" is any nucleotide; "R" is a nucleotide selected from A or G; "H" is a nucleotide selected from C, T or A; "D" is a nucleotide selected from G, T or A; "Y" is a nucleotide selected from C or T; "V" is a nucleotide selected from G, C or A and "W" is a nucleotide selected from T or A.

Particularly, the aptamer of the invention is any one of the aptamers Apt-HCVcore_01 (SEQ ID NO: 10), Apt-HCVcore_02 (SEQ ID NO: 11), Apt-HCVcore_03 (SEQ ID NO: 12), Apt-HCVcore_04 (SEQ ID NO: 13), Apt-HCVcore_05 (SEQ ID NO: 14), Apt-HCVcore_06 (SEQ ID NO: 15), Apt-HCVcore_07 (SEQ ID NO: 16) and Apt-HCVcore_08 (SEQ ID NO: 17).

So, in other even more preferred embodiment of the invention, the aptamer comprises or consists on the nucleotide sequence selected from the list consisting of: SEQ ID NO: 10, SEQ ID NO: 11, SEQ ID NO: 12, SEQ ID NO: 13, SEQ ID NO: 14, SEQ ID NO: 15, SEQ ID NO: 16 and SEQ ID NO: 17.

Particularly, the aptamer Apt-HCVcore_01 (SEQ ID NO: 10) also recognizes the region between 1 to 122 amino acids of the HCV core protein of the genotype 2 (SEQ ID NO: 43), of the core protein of the genotype 3 (SEQ ID NO: 44) or of the core protein of the genotype 4 (SEQ ID NO: 45).

As described above, the aptamer of the invention may be the complementary reverse sequence of nucleotide sequences SEQ ID NO: 10, SEQ ID NO: 11, SEQ ID NO: 12, SEQ ID NO: 13, SEQ ID NO: 14, SEQ ID NO: 15, SEQ ID NO: 16 or SEQ ID NO: 17.

So, in other preferred embodiment of the invention, the aptamer comprises or consists on the nucleotide sequence selected from the list consisting of: SEQ ID NO: 30, SEQ ID NO: 31, SEQ ID NO: 32, SEQ ID NO: 33, SEQ ID NO: 34, SEQ ID NO: 35, SEQ ID NO: 36 and SEQ ID NO: 37

Particularly, the aptamers Apt-HCVcore_09 to Apt-HCVcore_11 (Table 1) share common flanking regions (the underlined sequence areas), wherein the 5'-end comprises the nucleotide sequence GCGGATGAAGACTGGTGT (SEQ ID NO: 48) and the 3'-end comprises the nucleotide sequence GCCCTAAATACGAGCAAC (SEQ ID NO: 49), producing a nucleotide consensus sequence of the aptamers Apt-HCVcore_09 to Apt-HCVcore_11, the nucleotide consensus sequence SEQ ID NO: 8. So, in other preferred embodiment of the invention, the aptamer comprises or consists on the nucleotide sequence SEQ ID NO: 8
SEQ ID NO:8 
GCGGATGAAGACTGGTGTKBMRYHHYYBYSBCWYKHWMYTMTHYKWHCHHTC
CVKYSBGCCCTAAATACGAGCAAC
 wherein letter "S" is a nucleotide selected from C or G; "M" is a nucleotide selected from A or C; "K" is a nucleotide selected from G or T; "R" is a nucleotide selected from A or G; "B" is a nucleotide selected from G, C or T; "H" is a nucleotide selected from C, T or A; "D" is a nucleotide selected from G, T or A; "Y" is a nucleotide selected from C or T and "W" is a nucleotide selected from T or A.

As described above, the aptamer of the invention may be the complementary reverse sequence of nucleotide sequences SEQ ID NO: 8. So, in other preferred embodiment of the invention, the aptamer comprises or consists on the nucleotide sequence SEQ ID NO: 28.
SEQ ID NO: 28 
GTTGCTCGTATTTAGGGCVSRMBGGADDGDWMRDAKARKWDMRWGVSRVRR
DDRYKVMACACCAGTCTTCATCCGC
 wherein letter "S" is a nucleotide selected from C or G; "M" is a nucleotide selected from A or C; "K" is a nucleotide selected from G or T; "R" is a nucleotide selected from A or G; "B" is a nucleotide selected from G, C or T; "H" is a nucleotide selected from C, T or A; "D" is a nucleotide selected from G, T or A; "Y" is a nucleotide selected from C or T; "W" is a nucleotide selected from T or A and "V" is a nucleotide selected from C, G or A.

Further, the common flanking regions (the underlined sequence areas) of the aptamer comprises the nucleotide consensus sequence SEQ ID NO: 8 which may comprise one more nucleotide at the 3'-end, obtaining a nucleotide consensus sequence with a 77 nucleotides length (SEQ ID NO: 9).

Thus, in other more preferred embodiment of the invention, the aptamer comprises or consists on the nucleotide sequence SEQ ID NO: 9
SEQ ID NO: 9 
GCGGATGAAGACTGGTGTKBMRYHHYYBYSBCWYKHWMYTMTHYKWHCHHTC
CVKYSBGCCCTAAATAGCGAGCAAC
 wherein letter "S" is a nucleotide selected from C or G; "M" is a nucleotide selected from A or C; "K" is a nucleotide selected from G or T; "R" is a nucleotide selected from A or G; "B" is a nucleotide selected from G, C or T; "H" is a nucleotide selected from C, T or A; "D" is a nucleotide selected from G, T or A; "Y" is a nucleotide selected from C or T and "W" is a nucleotide selected from T or A.

As described above, the aptamer of the invention may be the complementary reverse sequence of nucleotide sequences SEQ ID NO: 9. So, in other preferred embodiment of the invention, the aptamer comprises or consists on the nucleotide sequence SEQ ID NO: 29.
SEQ ID NO: 29 
GTTGCTCGCTATTTAGGGCVSRMBGGADDGDWMRDAKARKWDMRWGVSRVR
RDDRYKVMACACCAGTCTTCATCCGC
 wherein letter "S" is a nucleotide selected from C or G; "M" is a nucleotide selected from A or C; "K" is a nucleotide selected from G or T; "R" is a nucleotide selected from A or G; "B" is a nucleotide selected from G, C or T; "H" is a nucleotide selected from C, T or A; "D" is a nucleotide selected from G, T or A; "Y" is a nucleotide selected from C or T; "W" is a nucleotide selected from T or A and "V" is a nucleotide selected from C, G or A.

Particularly, the aptamer of the invention is any one of the aptamers Apt-HCVcore_09 (SEQ ID NO: 18), Apt-HCVcore_10 (SEQ ID NO: 19) or Apt-HCVcore_11 (SEQ ID NO: 20).

In other even more preferred embodiment of the invention, the aptamer comprises or consists on the nucleotide sequence selected from the list consisting of: SEQ ID NO: 18, SEQ ID NO: 19 and SEQ ID NO: 20.

As described above, the aptamer of the invention may be the complementary reverse sequence of nucleotide sequences SEQ ID NO: 18, SEQ ID NO: 19 and SEQ ID NO: 20. So, in other preferred embodiment of the invention, the aptamer comprises or consists on the nucleotide sequence selected from the list consisting of: SEQ ID NO: 38, SEQ ID NO: 39 and SEQ ID NO: 40.

In the scope of the present invention, the aptamer of the invention can be an aptamer derivate. The term "aptamer derivate" as used herein refers to RNA or DNA aptamers, wherein at least one of the nucleic acids of said aptamer has a chemical modification. The person with average skill in the field may find that the introduction of chemical modifications which do not alter the nucleotide sequence and the spatial arrangement of the nitrogenous bases does not change the functionality of the molecule of the aptamers described in the present invention, which mainly depends on the sequence complementarity principle between nucleobases (to maintain its three-dimensional structure in solution) and the non-covalent interactions established between the folded aptamer and its target molecule (HCV core protein in the present invention). However, such chemical modifications have a positive effect because they increase the stability and the half-life of the aptamer derivate. The increase in stability and resistance to degradation of the aptamer results in an increase in its availability (in a buffer, in biological fluids or intracellularly) and, thus, in its overall efficiency.

By way of example and without limitation, this modification may be useful to increase resistance to degradation by nuclease enzymes (DNases or RNases) in biological media. Examples of modifications in nucleotides, that produce such increase in resistance to enzymatic degradation comprise a substituent in the sugar moiety (deoxyribose or ribose) of one or more nucleotides, include, without limitation a: fluorine atom, an amino group, a methyl group, a methoxy group and any combination thereof. Additionally, the 5'-end and/or the 3'-end of the aptamer can be chemically modified to avoid or reduce the action of exonuclease enzymes, by introducing a molecule selected, without limitation, among polyethylene glycol (PEG), cap structures (m7GpppG or GpppG), trimethoxystilbene, pyrene, inverted deoxythymidine bases and dideoxynucleotides, 8-oxoguanine, 8-oxoadenine and cholesterol adducts.

By way of example and without limitation, this modification may be useful to immobilize or functionalize the aptamer in the construction of biosensors. Examples of modifications for immobilizing or functionalizing the aptamer include, without limitation to, a thiol group, an amino group, a carboxyl group, an epoxy group, and any combination thereof.

In another preferred embodiment, the aptamer of the invention may be a functionally equivalent variant of any of the aptamers already defined by any of the above related nucleotide sequences with a capability of binding specifically to the HCV core protein of at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, or at least 100% of the capability of specifically binding to the core protein of the aptamer of sequences SEQ ID NO: 1 to SEQ ID NO: 40.

As it is used herein, the term "functionally equivalent variant" refers to aptamers with sequences substantially similar to SEQ ID NO: 1 to SEQ ID NO: 40 maintaining the capability of specifically binding to the core protein. A functionally equivalent variant of the aptamer of the invention can be a nucleic acid sequence derived from one of the sequences SEQ ID NO: 1 to SEQ ID NO: 40 comprising the addition, substitution or modification of one or more nucleotides.

By way of illustration, functionally equivalent variants of the aptamer of the invention include sequences comprising the addition of 1 nucleotide, 2 nucleotides, 3 nucleotides, 4 nucleotides, 5 nucleotides, 10 nucleotides, 15 nucleotides, 20 nucleotides, 25 nucleotides, 30 nucleotides, 35 nucleotides, 40 nucleotides, 45 nucleotides, 50 nucleotides, 60 nucleotides, 70 nucleotides, 80 nucleotides, 90 nucleotides, 100 nucleotides, 150 nucleotides, 200 nucleotides, at least 500 nucleotides, at least 1000 nucleotides or more at the 5' end of the sequences SEQ ID NO: 1 to SEQ ID NO: 40, and/or comprising the addition of 1 nucleotide, 2 nucleotides, 3 nucleotides, 4 nucleotides, 5 nucleotides, 10 nucleotides, 15 nucleotides, 20 nucleotides, 25 nucleotides, 30 nucleotides, 35 nucleotides, 40 nucleotides, 45 nucleotides, 50 nucleotides, 60 nucleotides, 70 nucleotides, 80 nucleotides, 90 nucleotides, 100 nucleotides, 150 nucleotides, 200 nucleotides, at least 500 nucleotides, at least 1000 nucleotides or more at the 3' end of the sequences SEQ ID NO: 1 to SEQ ID NO: 40 and maintaining a capability of specifically binding to the core protein of at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, or at least 100% of the capability of specifically binding to the core protein.

Also, in a preferred embodiment, the functionally equivalent variant of the aptamer of the invention comprises a nucleotide sequence with at least 85%, at least 90%, at least 91%, at least 92%, at least 93%, at least 94%, at least 95%, at least 96%, at least 97%, at least 98%o or at least 99% identity with sequences SEQ ID NO: 1 to SEQ ID NO: 40. The term "% identity" has been previously defined and said definition is equally valid for this embodiment of the present invention.

The aptamer of the invention can be included in a genetic construct. Therefore, other aspect of the invention relates to a genetic construct that comprises the aptamer of the invention, hereinafter the "genetic construct of the invention".

The term "genetic construct" used herein refers to a DNA or RNA sequence useful for the synthesis by transcription, either *in vitro* or intracellular, of the aptamer of the invention, characterized in that it comprises a nucleotide sequence encoding the nucleotide sequence of the aptamer of the present invention and optionally includes regulatory sequences, such a promotor or an initiation and termination signal.

The aptamer or the genetic construct of the present invention can be included in a vector. So, other aspect of the invention relates to a vector comprising the aptamer or the genetic construct of the invention, hereinafter the "vector of the invention".

The term "vector", as used herein, refers to a construct capable of delivering, and optionally expressing, one or more aptamer of the invention into a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells. The vectors can be stable and can be self-replicating. There are no limitations regarding the type of vector that can be used. The vector can be a cloning vector, suitable for propagation and for obtaining polynucleotides, gene constructs or expression vectors incorporated to several heterologous organisms. Suitable vectors include prokaryotic expression vectors (e.g. pUC18, pUC19, Bluescript and their derivatives), mpl8, mpl9, pBR322, pMB9, CoIEI, pCRI, RP4, phages and shuttle vectors (e.g. pSA3 and pAT28), and eukaryotic expression vectors based on viral vectors (e.g. adenoviruses, adeno- associated viruses as well as retroviruses and lentiviruses), as well as non-viral vectors such as pSilencer 4.1-CMV (Ambion^{®}, Life Technologies Corp., Carslbad, CA, US), pcDNA3, pcDNA3.1/hyg pHCMV/Zeo, pCR3.1, pEFI/His, pIND/GS, pRc/HCMV2, pSV40/Zeo2, pTRACER-HCMV, pUB6/V5-His, pVAXI, pZeoSV2, pCI, pSVL and pKSV-10, pBPV-I, pML2d and pTDTI.

As a person skilled in the art knows, the aptamer or the genetic construct or the vector of the invention can be included in a host cell. So, other aspect of the invention relates to a host cell comprising the aptamer or the genetic construct or the vector of the invention, hereinafter the "host cell of the invention".

As a person skilled in the art knows, the aptamer or the genetic construct or the vector of the invention can be included in a composition. So, other aspect of the invention relates to a composition comprising the aptamer or the genetic construct or the vector of the invention, hereinafter the "composition of the invention".

In a preferred embodiment, the composition of the invention is a pharmaceutical composition, hereinafter the "pharmaceutical composition of the invention".

As used in the present description, the term "pharmaceutical composition" refers to any substance used for the diagnosis, prevention, alleviation, treatment or cure of a disease in a human, particularly hepatitis C disease. The pharmaceutical composition of the invention can be used alone or in combination with other pharmaceutical compositions.

In other preferred embodiment, the pharmaceutical composition of the invention further comprises a pharmaceutically acceptable carrier or excipient.

The term "pharmaceutically acceptable excipient" refers to a substance which helps the absorption of the pharmaceutical composition comprising the aptamer of the invention, stabilizes said pharmaceutical composition or helps in the manufacture thereof in the sense of giving it consistency, form, flavor or any other specific functional characteristic. Thus, excipients could have the function of keeping the ingredients bound together (such as for example starches, sugars or celluloses), an encapsulating function (such as for example lipids, fatty acids or other amphiphilic molecules that can form vesicles or liposomes), a sweetening function, a colorant function, a protection function (such as for example isolating it from the air and/or moisture), a filler function for a tablet, capsule or any other form of formulation, such as for example dibasic calcium phosphate, a disintegrating function to facilitate the dissolution of the components and their absorption, without excluding other types of excipients not mentioned in this paragraph.

A "pharmaceutically acceptable carrier" (or "pharmacologically acceptable") refers to any substance, or combination of substances, known in the pharmaceutical sector, used in the manufacture of pharmaceutical forms of administration and includes, but is not limited to, solids, liquids, solvents or surfactants. The carrier can be an inert substance or have a similar action to any of the compounds of the present invention, with the function of facilitating the incorporation of the drug as well as other compounds, allowing for an improved dosage and administration or providing consistency and form to the pharmaceutical composition. When the dosage form is liquid, the carrier is the diluent. The term "pharmacologically acceptable" refers to the fact that the compound referred to is allowed and evaluated so that it does not cause harm to the organisms to which it is administered.

The pharmaceutical composition of the invention can be administered through any route of administration, and as such, said composition shall be formulated in the pharmaceutical form suitable to the chosen route of administration. Thus, the pharmaceutical composition of the invention can be administered by oral, nasal, ocular, topical, intradermic, intracranial, intraperitoneal or intravenous route.

The aptamers of the invention can interfere with HCV replication cycle. For this, it is possible the application of aptamers as therapeutic molecules in viral infections, particularly in HCV infection.

So, other aspect of the invention relates to the aptamer, the genetic construct or the vector, or the composition of the invention for use as a medicament. Alternatively, the present invention relates to the use of the aptamer, the genetic construct or the vector, or the composition of the invention, in the manufacture of a medicament. Hereinafter, the "first medical use of the invention".

Other aspect of the invention relates to the aptamer, the genetic construct or the vector, or the composition of the invention for use in the treatment or diagnosis of hepatitis C. Alternatively, the present invention relates to the use of the aptamer, the genetic construct or the vector, or the composition of the invention, in the manufacture of a medicament for the treatment or diagnosis of hepatitis C. Hereinafter, the "second medical use of the invention".

As used herein, the term "treating" (or "treat" or "treatment") refers to processes involving a slowing, interrupting, arresting, controlling, stopping, reducing, or reversing the progression or severity of an existing symptom, disorder, condition, or disease, but does not necessarily involve a total elimination of all disease-related symptoms, conditions, or disorders. The treatment of a disorder or disease may, for example, lead to a halt in the progression of the disorder or disease (e.g., no deterioration of symptoms in the subject/patient) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may, e.g., lead to a halt in the progression of the disorder or disease or a delay in the progression of the disorder or disease. Such a partial or complete response may be followed by a relapse. It is to be understood that a subject/patient may experience a broad range of responses to a treatment (such as the exemplary responses as described herein above). In the present invention, the condition or disorder to be treated is hepatitis C.

Biosensors have been developed for use in several fields of medical care, drug discovery and clinical essays, among others. In general, biosensors use probe biomolecules or compounds that specifically capture and identify other target biomolecules (such as structural proteins, enzymes, antibodies, DNA or RNA), or even microorganisms and eukaryotic cells. Biosensors convert the probe-target interaction into a measurable physical or chemical signal, which can involve, among other options, optical, electrical, electrochemical, thermal or mechanical principles. Among the molecular probes used in biosensors, aptamers offer a number of advantages over antibodies, thus a number of aptamer-based biosensors, also called "aptasensors" have been developed.

Thus, other aspect of the present invention relates to a biosensor comprising the aptamer, the genetic construct or the vector of the invention, hereinafter the "biosensor of the invention".

As used herein, the term "biosensor" refers to a device that detects a target (biological or chemical) substance, condition, or reaction through the use of a biomolecule and transmits information about the biological or chemical substance, condition, or reaction as a signal. In the present invention the biosensor recognizes or detects HCV either *in vitro* (in a buffer or salt solution with the required composition) or in biological fluids (such as blood, plasma, urine, serum, saliva, mucus, interstitial fluid, amniotic fluid, lymphatic fluid, pericardial fluid, peritoneal fluid, and tears), thanks to the interaction among the aptamer probes and HCV core protein.

Examples of biosensor include, without limitation to, electrochemical biosensor, optical biosensor or nanomechanical biosensor.

In a preferred embodiment of the invention, the biosensor of the present invention comprises an electrochemical biosensor, which comprises an electrode having a surface onto which a biomolecule, particularly the aptamer of the invention, is immobilized and which transmits information about a biological or chemical substance, condition, or reaction as an electrical signal. The aptamer may be immobilized on the surface of the biosensor by direct chemical bonding or by indirect bonding through a polymer film that is applied to the surface of the sensor.

As a person skilled in the art knows, the aptamer (or the genetic construct or the vector or the composition of the invention) or the biosensor of the invention can be used for *in vitro* diagnosis of hepatitis C.

So, other aspect of the present invention relates to the use *in vitro* of the aptamer, the genetic construct, the vector, the composition or the biosensor of the invention for the diagnosis of hepatitis C.

The term "diagnosing" as used herein refers to the procedure by which a certain disease, syndrome, or any health-disease condition is identified through the analysis of a series of clinical parameters or symptoms characteristic of that disease, and which distinguish it from other diseases with similar clinical presentation. In the present invention the disease to be identified is hepatitis C.

As describe above, the aptamer of the invention specifically binds to core protein of HCV, which can be used in a method *in vitro* for detecting or diagnosis of hepatitis C in a subject/patient.

Thus, other aspect of the present invention relates to a method for the *in vitro* detection or diagnosis of hepatitis C infection wherein the method comprises:
(i) contacting an isolated biological sample from a subject with the aptamer that binds to HCV core protein, i.e. the aptamer of the invention, or the biosensor of the invention, and
(ii) detecting the binding of the aptamer to HCV core protein,
wherein the binding between the aptamer and HCV core protein present in the biological sample is indicative of the presence of hepatitis C infection in said subject/patient.

The terms "hepatitis C virus", "HCV core protein" and "aptamer" have been previously defined and said definitions are equally valid for the present aspects as well as its embodiments.

As used herein, the term "subject" or "patient" refers to human and non-human animals, such as veterinary subjects. The term "non-human animal" includes all vertebrates, e.g., mammals and non-mammals, such as non-human primates, mice, rabbits, sheep, dog, cat, horse, cow, chickens, amphibians, and reptiles. In a particular embodiment, the subject is a human, man or woman of any age.

The term "sample" or "isolated sample" as used herein refers to part, fraction, portion, or sub-volume considered to be representative of a larger volume of the material/object/volume to be sampled. In a preferred embodiment of the method of the invention the sample is a biological sample selected from a list consisting of: blood, plasma, urine, serum, saliva, mucus, interstitial fluid, amniotic fluid, lymphatic fluid, pericardial fluid, peritoneal fluid or tears.

It is a routine practice, for the person skilled in the art, the methods or techniques for detecting the binding of the aptamer to HCV core protein. Examples of techniques for detecting the interaction or the binding between aptamer with HCV core protein include, without limitation to, ELISA and ELONA (in both cases, combined to either colorimetric or (RT)qPCR amplification), lateral flow-based techniques, nanoparticle-based techniques, microarray-based techniques, microfluidic-based techniques, and the use of different kinds of biosensors previously explained.

In other preferred embodiment of the invention, since the aptamer of the invention is not by itself a detectable molecule, the step of detection may imply an indirect detection through a second detectable molecule or reagent which binds specifically to the aptamer. Said reagent can be detected by means of fluorimetry or colorimetry using apparatuses suitable for the type of reagents and the type of sample, which are known by the person skilled in the art. By way of example, the sample with the aptamer bound to HCV core protein is incubated with an antibody specific for the aptamer that is conjugated with an enzyme, in conditions similar to the conditions of incubation with the aptamer, and the HCV core-antibody complexes are detected thanks to the addition of a substrate that is converted by the enzyme into a detectable product, for example, by means of fluorimetry in a fluorescence microscope or by colorimetry in a spectrophotometer. Alternatively, detection can be done in an analogous manner by means of the use of a probe specific for the aptamer suitably labeled with a detectable reagent.

In other more preferred embodiment of the invention, the reagent is an enzyme. The term "enzyme", in the context of the present invention, refers to a protein working as a highly selective catalyst, accelerating both the speed and the specificity of the metabolic reaction for which it is specific. Examples of enzymes suitable for the invention include, without limitation to, horseradish peroxidase (HRP) and alkaline phosphatase. As the person skilled in the art will understand, the enzymes suitable for use in the present invention are indirectly detectable as a result of their capability of catalyzing the modification of a substrate in a compound detectable by colorimetry, chemiluminescence or fluorimetry. Examples of suitable substrates include, without limitation to, p-Nitrophenyl phosphate (PNPP), 2,2'-azinobis[3-ethylbenzothiazolin-6-sulfonic acid] (ABTS), o-phenylenediamine (OPD) and 3,3',5,5'-tetramethylbenzidine (TMB).

Bioluminescent proteins or photoproteins are a particular case of oxidative enzymes capable of carrying out a chemical reaction of their specific prosthetic groups, resulting in light emission without requiring prior excitation. Non-limiting examples of bioluminescent proteins include firefly luciferase, Renilla luciferase and aequorin.

In other more preferred embodiment, the reagent is a protein, preferably is a fluorescent protein. The term "fluorescent protein", in the context of the present invention, refers to a protein with the capability of emitting light when it is excited at a wavelength suitable for excitation. Non-limiting examples of fluorescent proteins that can be used in the complex of the invention include, without limitation, GFP, GFPuv, BFP, CFP, YFP, EBFP2, mCerulean, mCerulean3, mVenus, mTurquoise, T-Sapphire, citrine, amFP486, zFP506, zFP538, drFP, DsRed, mCherry, dTomate, mTFP1, TagRFP-T, mKO2, mRuby, mKate, mAmetrine, REACh, R-phycoerythrin (R-PE) and Allophycocyanin (APC).

### DESCRIPTION OF THE DRAWINGS

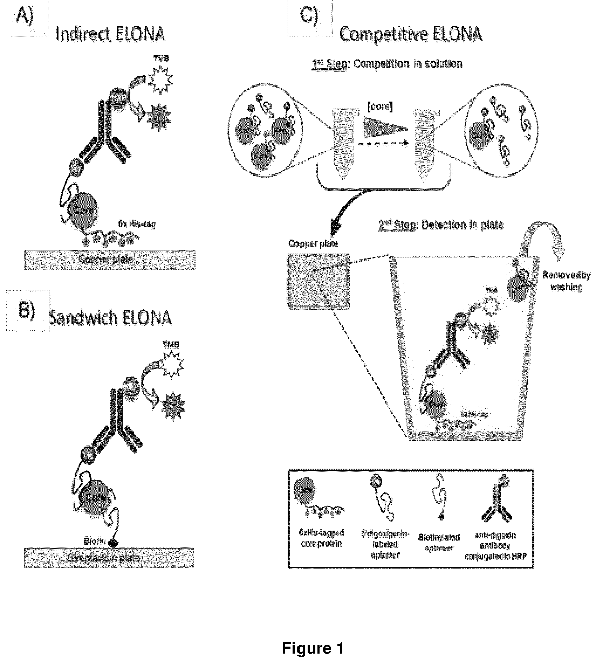

**Figure 1****.** Scheme of three different colorimetric ELONA formats used in this invention to detect HCV core protein (represented by the protein G1-122 in this drawing). Aptamer-based protein detection was performed by means of an indirect ELONA (A), a sandwich ELONA (B) and competitive ELONA (C) to analyze the affinity and specificity of the aptamers against the target.

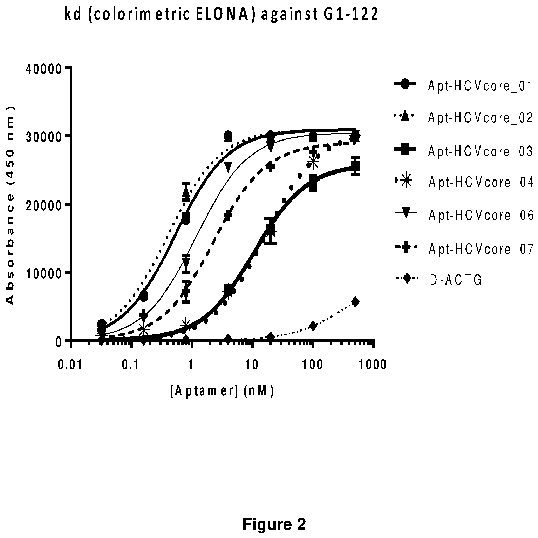

**Figure 2****.** Characterization of the binding affinities of individual ssDNA aptamers selected against core G1-122, by means of colorimetric ELONA. The affinity curves obtained for the six individual aptamers represented in this graph show that the best fit curve corresponds to a one site-specific binding model (with R² values of 0.99) in all cases, from which the Kd can be derived. Each data point represents the mean ± SD (n = 3). In this set of experiments, the obtained values are: Apt-HCVcore_01, Kd = 0.5 nM; Apt-HCVcore_02, Kd = 0.4 nM; Apt-HCVcore_03, Kd = 20.0 nM; Apt-HCVcore_04, Kd = 16.2 nM; Apt-HCVcore_06, Kd = 1.2 nM; Apt-HCVcore_07, Kd = 2.3 nM.

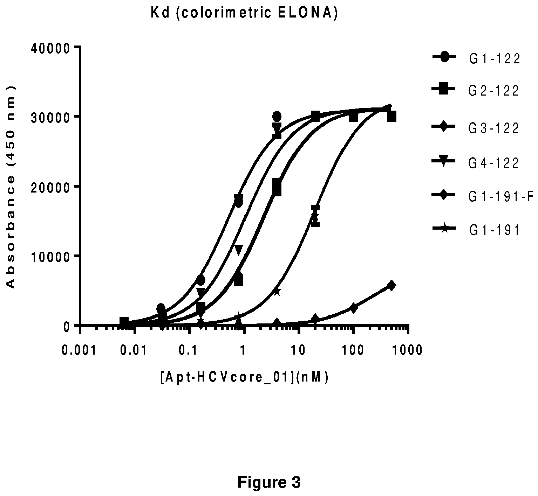

**Figure 3****.** Colorimetric ELONA of the ssDNA aptamer Apt-HCVcore_01 against six HCV core protein variants. The affinity curves obtained show that the best fit curve corresponds to a one site-specific binding model (with R² values of 0.99) in all cases, from which the Kd can be derived. Each data point represents the mean ± SD (n = 3).

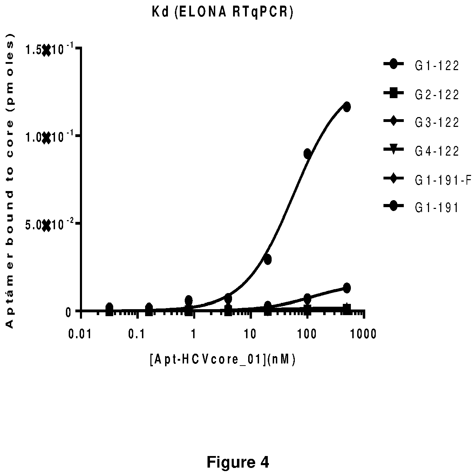

**Figure 4****.** Binding affinities of the RNA version of Apt-HCVcore_01 against six variants of HCV core protein, quantified by ELONA-RTqPCR. The affinity curves obtained show that the best fit curve corresponds to a one site-specific binding model (with R² values of 0.99) in all cases, from which the Kd can be derived. Each data point represents the mean ± SD (n = 3).

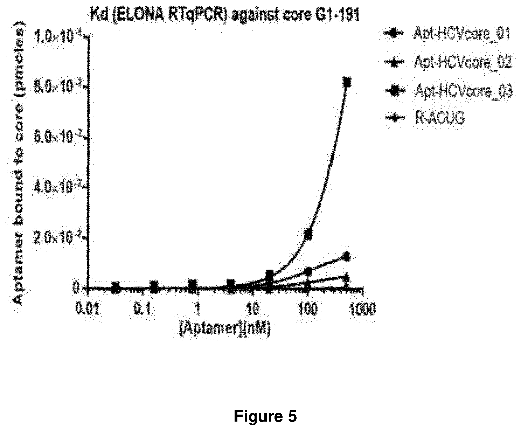

**Figure 5****.** Binding affinities of the RNA versions of aptamers Apt-HCVcore_01, Apt-HCVcore_02 and Apt-HCVcore_03 against the G1-191 core protein, quantified by ELONA-RT-qPCR. The affinity curves obtained show that the best fit curve corresponds to a one site-specific binding model (with R² values of 0.99) in all cases, from which the Kd can be derived. In parallel, the RNA molecule used as the negative control (termed R-ACUG, SEQ ID NO: 42) can only be adjusted to a linear regression model, thus revealing its unspecific binding to core protein. Each data point represents the mean ± SD (n = 3).

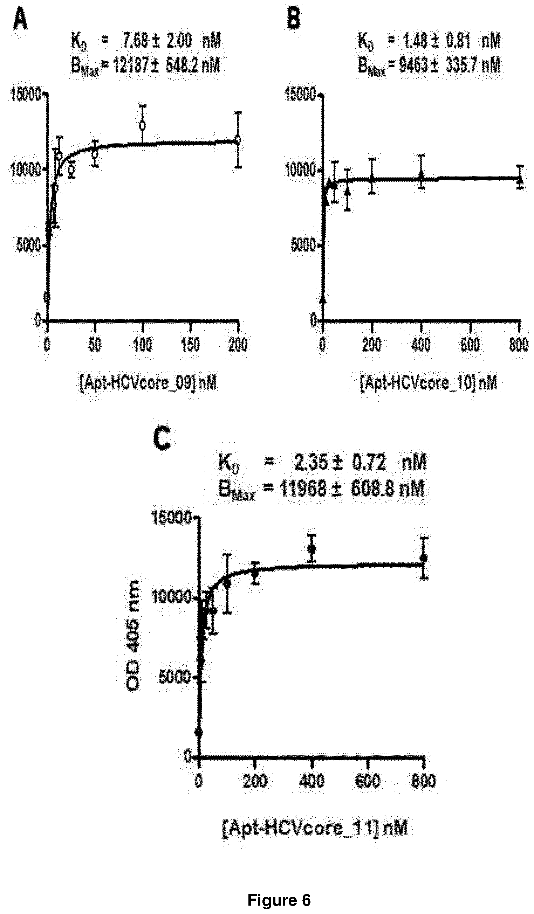

**Figure 6****.** Binding affinity of ssDNA aptamers Apt-HCVcore_09 (A), Apt-HCVcore_10 (B) and Apt-HCVcore_11 (C) for G1-122 core protein. In these experiments, anti-digoxigenin antibody was added and revealed with ABTS solution at 405 nm. Each data point represents the mean ± SD (n = 3).

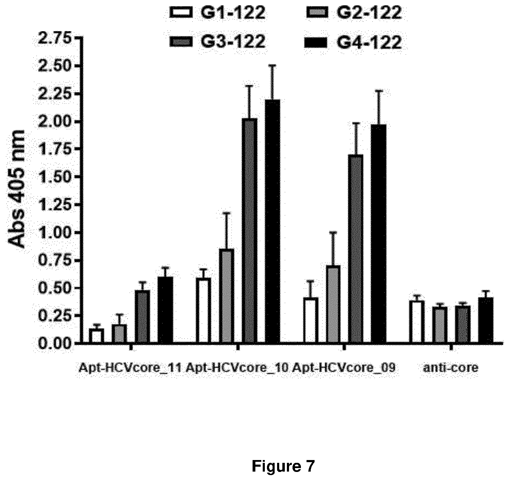

**Figure 7****.** Binding affinity of ssDNA aptamers Apt-HCVcore_09, Apt-HCVcore_10 and Apt-HCVcore_11 for G1-122, G2-122, G3-122 and G4-122 core proteins. In these experiments, anti-digoxigenin antibody was added and revealed with ABTS solution at 405 nm. The results using an anti-HCVcore antibody as a reference are also shown. Each data point represents the mean ± SD (n = 3).

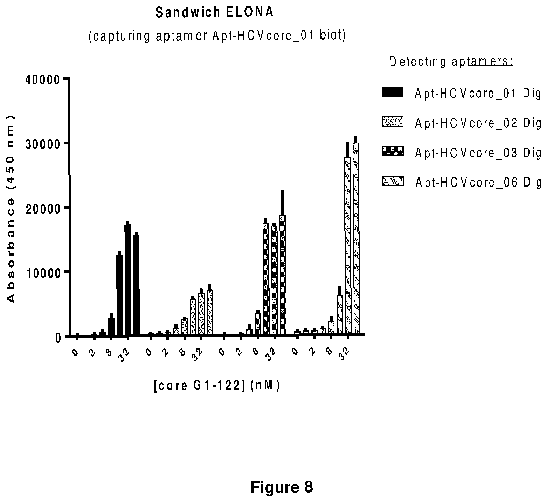

**Figure 8****.** Colorimetric sandwich ELONA using a biotinylated version of Apt-HCVcore_01 as capturing aptamer, and the digoxigenin-labeled ssDNA aptamers Apt-HCVcore_01, Apt-HCVcore_02, Apt-HCVcore_03 or Apt-HCVcore_06 as detecting aptamers. In this assay, every aptamer bound to HCV core protein G1-122 (with the exception of Apt-HCVcore_02) shows a lower LoD of the protein when compared to direct assays, thus suggesting the partial overlapping of their binding sites on HCV core protein. In turn, it is observed that the aptamer Apt-HCVcore_01 itself contains different binding sites to core. Each data point represents the mean ± SD (n = 3).

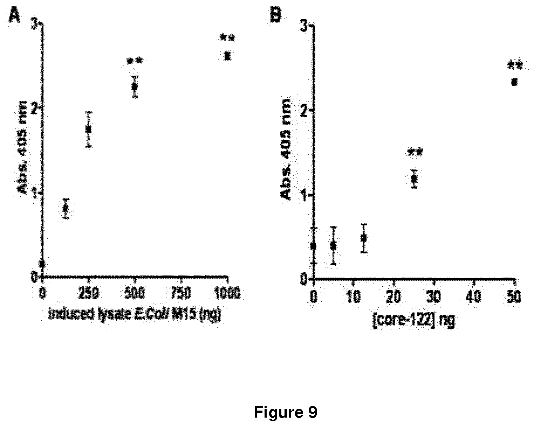

**Figure 9****.** Colorimetric sandwich ELONA using a biotinylated version of Apt-HCVcore_09 as capturing aptamer in combination with the digoxigenin-labeled ssDNA aptamer Apt-HCVcore_10. The target HCV core proteins were either G1-191 expressed in *E. coli* M15 strain (A) or G1-122 supplemented in human plasma from an uninfected subject (B). Each data point represents the mean ± SD (n = 3). Statistical significance was expressed as ** p<0.01.

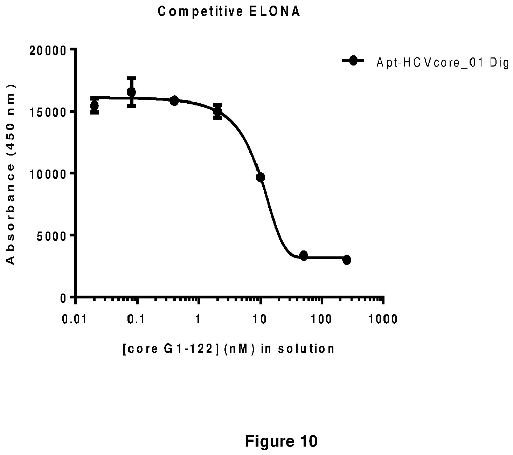

**Figure 10****.** Competitive ELONA of ssDNA aptamer Apt-HCVcore_01 against G1-122 core protein. Note that only concentrations down to 0.01 nM are depicted. Each data point represents the mean ± SD (n = 3).

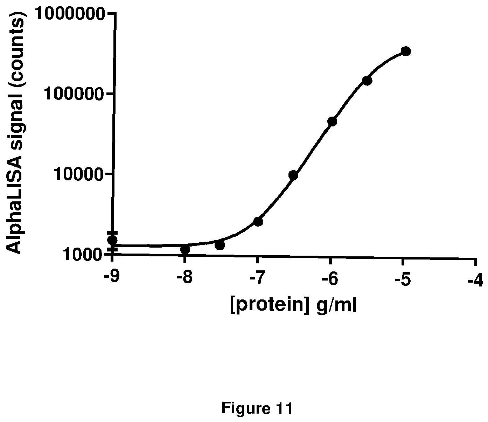

**Figure 11****.** AlphaLISA assay: dilution curve of the His-tagged HCV core protein to determine the LoD and dynamic range of the assay, using 30 nM of the biotinylated aptamer Apt-HCVcore_10. AlphaLISA counts are plotted versus the concentration of HCV core protein, according to a nonlinear regression and using the 4-parameter logistic equation (sigmoidal dose-response curve with variable slope) and a 1/Y2 data weighting. The LoD obtained is 0.1 µg/ml.

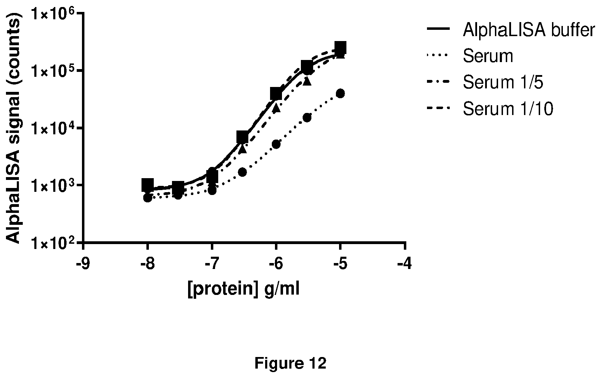

**Figure 12****.** AlphaLISA assay: dilution curve in human serum from an uninfected patient. A full dilution curve of HCV core protein, using 30 nM of biotinylated Apt-HCVcore_10, was performed in four media: AlphaLISA buffer, human serum, human serum diluted 1/5, and human serum diluted 1/10. The results show that undiluted serum interferes with the assay and increases its LoD, while a 1/5 dilution of the serum significantly avoids such an interference.

### EXAMPLES

### 1. In vitro selection of aptamers against HCV core protein

The inventors have developed specific aptamers against molecular targets of HCV, specifically against the protein of the viral capsid (core protein). Aptamers are oligomers of single-stranded nucleic acids (ssDNA or RNA), obtained using *in vitro* selection systems which start from combinatorial populations of nucleic acids. The three-dimensional structure of aptamers enables them to non-covalently bind with high affinity and specificity to the desired target molecule.

Specifically, 74 to 77 nucleotides long, ssDNA and RNA aptamers were selected *in vitro* after following a methodology analogous to that previously described by the inventors for a different target (Miguel Moreno et al., Molecules 2019; 24:1213). In the current invention, six variants of the HCV core protein were used as target molecules. Two of them contained the full-length sequence of HCV core (amino acids 1 to 191), belonging to genotype 1: a C-terminal biotin-tagged, fusion protein with β-galactosidase (termed G1-191-F), and an N-terminal 6xHis-tagged protein expressed in a baculovirus-insect systems (termed G1-191).

Additionally, the N-terminal 6xHis-tagged hydrophilic domain (amino acids 1 to 122) of HCV core expressed in a baculovirus-insect system and belonging to the four main genotypes of the virus were also used as targets (termed G1-122, G2-122, G3-122 and G4-122, respectively). In this work, up to 14 rounds of *in vitro* selection (including counter-selection steps to increase the specificity of the process) were used to obtain ssDNA and RNA aptamers.

### 2. Characterization of the in vitro selected aptamers against HCV core protein

The characterisation of the affinity and specificity of the aptamers for the targets thereof was performed using different techniques, among them those based on ELONA (a system analogous to ELISA but using aptamers instead of antibodies). The functional analysis of ssDNA aptamers involved ELONA coupled to colorimetric methods. With that aim, different concentrations of HCV core protein were immobilized onto chemically modified multiwell plates, and a 5' digoxigenin-labelled version of the ssDNA aptamer was bound to them. Then, the use of an anti-digoxigenin antibody labelled with horseradish peroxidase (HRP) allowed the detection of a colorimetric reaction which is proportional to the amount of aptamer bound to HCV core. Using this basic methodology, three colorimetric ELONA formats were assayed, as depicted in Figure 1: indirect ELONA, sandwich ELONA and competitive ELONA. In turn, ELONA coupled to real time quantitative RT-PCR (using SYBR Green as dye) allowed analysing the performance of RNA aptamers. In both cases, the corresponding calibration curves were previously obtained, with which the affinity of each aptamer (expressed as the dissociation constant, Kd) was quantified.

As a negative control, the ssDNA oligonucleotide termed D-ACTG (SEQ ID NO: 50) was used, which contains 10 repetitions of the tetranucleotide ACTG at its 40 nucleotides-long central region, the complete sequence thereof being (with 76 nucleotides, in the 5'-3' direction):
SEQ ID NO: 50

GCGGATCCAGACTGGTGTACTGACTGACTGACTGACTGACTGACTGACT
GACTGGCCCTAAAGACAAGCTTC

For RNA aptamers, the negative control used was the oligonucleotide termed R-ACUG (SEQ ID NO: 51), which contains 10 repetitions of the tetranucleotide ACUG at its 40 nucleotides-long central region, the complete sequence thereof being (with 76 nucleotides, in the 5'-3' direction):
SEQ ID NO: 51

GCGGAUCCAGACUGGUGUACUGACUGACUGACUGACUGACUGACUGA
CUGACUGGCCCUAAAGACAAGCUUC

The inventors performed comparative analyses of the results obtained, thus enabling the identification of the 11 aptamers which showed higher affinity for the HCV core protein. They were 74 to 77 nucleotides long and their affinity constant values (Kd) were comprised between 0.4 and 197.2 nM (Table 1).

**Table 1. Aptamers with higher affinity for the HCV core protein.**

| **Aptamer** | **Sequence (5' - 3')** | **Length (nt)** | **HCV core affinity (average Kd, nM)** | **SEQ ID** |
|---|---|---|---|---|
| Apt-HCVcore_01 | GCGGATCCAGACTGGTGTGCCGTATC CCTCCCTTGTAATTATTTGTTCCATCC GTTCCGCCCTAAAGACAAGCTTC | 76 | 0.82 | SEQ ID NO: 10 |
| Apt-HCVcore_02 | GCGGATCCAGACTGGTGTGTGCGGG TGGGTGGGTGGTTTGTTCTGCTGTGT TGTTGTGGCCCTAAAGACAAGCTTC | 76 | 0.40 | SEQ ID NO: 11 |
| Apt-HCVcore_03 | GCGGATCCAGACTGGTGTATCTTTTG CATGACGACTATTTACTAACTTTTCTC TAGGCCCTAAAGACAAGCTTC | 74 | 20.03 | SEQ ID NO: 12 |
| Apt-HCVcore_04 | GCGGATCCAGACTGGTGTAGTTCGG GCTTATATATACGAACCCTTAGTAATC CATGGGCCCTAAAGACAAGCTTC | 75 | 11.14 | SEQ ID NO: 13 |
| Apt-HCVcore_05 | GCGGATCCAGACTGGTGTGGGGTTTC TGTGTACCCTGGCTGCAGACGTATAT TTTCGGGCCCTAAAGACAAGCTTC | 76 | 11.53 | SEQ ID NO: 14 |
| Apt-HCVcore_06 | GCGGATCCAGACTGGTGTGGTGGTAA TTTTGTGTCTGGTTTGTTCTCTTTCTC GTTGTGCCCTAAAGACAAGCTTC | 76 | 1.18 | SEQ ID NO: 15 |
| Apt-HCVcore_07 | GCGGATCCAGACTGGTGTGTGGAATG TGTGGTGTTCACGGTGTAATTCTTTTT TTCGGCCCTAAAGACAAGCTTC | 75 | 2.29 | SEQ ID NO: 16 |
| Apt-HCVcore_08 | GCGGATCCAGACTGGTGTGTAACCCT TGCCGCTCTCTACTCTACGTACACTC CCTCGGGCCCTAAAGACAAGCTTC | 76 | 6.21 | SEQ ID NO: 17 |
| Apt-HCVcore_09 | GCGGATGAAGACTGGTGTGCCGTATC CCTCCCTTGTAATTATTTGTTCCATCC GTTCCGCCCTAAATACGAGCAAC | 76 | 2.35 | SEQ ID NO: 18 |
| Apt-HCVcore_10 | GCGGATGAAGACTGGTGTGTAACCCT TGCCGCTCTCTACTCTACGTACACTC CCTCGGGCCCTAAATAGCGAGCAAC | 77 | 1.48 | SEQ ID NO: 19 |
| Apt-HCVcore_11 | GCGGATGAAGACTGGTGTTGCACTAC CTTGTCATGAACTTCTCCTACCTTTCC AGCCTGCCCTAAATACGAGCAAC | 76 | 7.68 | SEQ ID NO: 20 |

Examples of the ELONA-based quantification of the Kd of different aptamers included in Table 1 are shown in Figures 2 to 7. One of the ssDNA aptamers that demonstrated high affinity for the HCV core protein in the nanomolar range was Apt-HCVcore_01 (Kd = 0.82 nM for G1-122, Kd = 2.4 nM for G2-122, Kd = 2.24 nM for G3-122, and Kd = 1.06 nM for G4-122; Figure 3).

### 3. Sandwich and competitive ELONA assays as diagnostic platforms for HCV.

Sandwich and competitive ELONA assays (see Figure 1) were developed to determine the functionality of the selected aptamers for the detection of HCV core protein, as well as to envisage aptamer-based diagnostic platforms of HCV. Figures 8 and 9 show relevant results obtained using the sandwich assay. In particular, the range of detection of HCV core by Apt-HCVcore_01, Apt-HCVcore_02, Apt-HCVcore_03 and Apt-HCVcore_06 as detecting aptamers (with a biotinylated version of Apt-HCVcore_01 as capturing aptamer) was found to be 3.91 to 62.5 nM, and the limit of detection (LoD) was 0.07, 0.15, 0.11 and 0.03 nM, respectively.

Regarding the competitive ELONA assay, Apt-HCVcore_01 was incubated with different concentrations of G1-122 in solution, and later on the free aptamer was bound to the immobilized core in a chemically modified multiwell plate (as schematized in Figure 1). The results showed that, as expected, higher concentrations of G1-122 in solution lead to less aptamer bound to the immobilized core in the plate, thus lowering the absorbance signal measured (Figure 10). The range of detection of HCV core was from 50 to 0.01 nM. The LoD was 2.5 nM, and the EC50 obtained was 7.2 ± 4.0 nM.

### 4. AlphaLISA assay

AlphaLISA was developed as an alternative assay to detect HCV core using aptamer-based systems. It offers a number of advantages over some of the methodologies described above, including the following: it avoids the need of washing steps, the protocol requires fewer steps and less sample amount, and it shows a broad dynamic range (Lucille Beaudet et al., Nature Methods 2008; 5:an8-an9).

In the experiments performed, a titration of the biotinylated aptamer Apt-HCVcore_10 (from 30 nM down to 0.3 nM) was carried out to find its optimal concentration in the assay. Such a titration curve used a fixed concentration of anti-His beads, two different concentrations of 6xHis-tagged G1-122 core protein and AlphaLISA buffer. To prevent any steric hindrance between the aptamer, protein and anti-His beads that could interfere with the binding of the aptamer to the protein, the aptamer and protein were incubated for 1 hour before the addition of both donor and acceptor beads.

As expected, the signal increased with higher concentrations of HCV core and Apt-HCVcore_10 (Table 2). At a concentration of 300 nM of aptamer the signal decreased because of saturation of the beads. Therefore, the optimal concentration of Apt-HCVcore_10 in the AlphaLISA assay was in the range of 30 to 100 nM.

**Table 2. Values obtained in the titration curve of AlphaLISA.**

| [Apt-HCVcore_10] | [HCV core] | | | | | | | |
|---|---|---|---|---|---|---|---|---|
| | 10 µg/ml | | 1 µg/ml | | 0.1 µg/ml | | 0 µg/ml | |
| 300 nM | 542739 | 527340 | 108618 | 120485 | 27701 | 28897 | 1772 | 1989 |
| 100 nM | 610884 | 628124 | 98884 | 106992 | 11909 | 11661 | 1264 | 1619 |
| 30 nM | 402864 | 387560 | 94997 | 111308 | 19088 | 18781 | 1221 | 1342 |
| 0 | 1487 | 1252 | 1088 | 1265 | 1265 | 975 | 1136 | 1284 |

Then, a dilution curve of the His-tagged HCV core protein was performed to determine the LoD and dynamic range of the assay, using 30 nM of the biotinylated aptamer Apt-HCVcore_10 (Figure 11).

Finally, to analyze the effect of human serum (from an uninfected patient) on the detection limit of the AlphaLISA assay, a full dilution curve of HCV core protein, using 30 nM of biotinylated Apt-HCVcore_10, was performed in four media: AlphaLISA buffer, human serum, human serum diluted 1/5, and human serum diluted 1/10 (Figure 12

## Claims

1. An aptamer which specifically binds to the region consisting of amino acids between 1 and 122 of the core protein of the hepatitis C virus (SEQ ID NO: 41), wherein the aptamer comprises a nucleotide sequence with at least 80% identity with SEQ ID NO: 1.

2. The aptamer according to claim 1 wherein the aptamer comprises the nucleotide sequence SEQ ID NO: 1.

3. The aptamer according to claims 1 or 2 wherein the aptamer comprises the nucleotide sequence SEQ ID NO: 2, SEQ ID NO: 3 or SEQ ID NO: 4.

4. The aptamer according to any one of claims 1 to 3 wherein the aptamer comprises the nucleotide sequence SEQ ID NO: 5 or SEQ ID NO: 8.

5. The aptamer according to claim 4 wherein the aptamer comprises the nucleotide sequence SEQ ID NO: 6, SEQ ID NO: 7 or SEQ ID NO: 9.

6. The aptamer according to claims 4 or 5 wherein the aptamer comprises a nucleotide sequence selected from the list consisting of: SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19 and SEQ ID NO: 20.

7. A genetic construct comprising the aptamer according to any one of claims 1 to 6.

8. A vector comprising the aptamer according to any one of claims 1 to 6 or the genetic construct according to claim 7.

9. A composition comprising the aptamer according to any of claims 1 to 6, or the genetic construct according to claim 7 or the vector according to claim 8, preferably wherein the composition is a pharmaceutical composition.

10. The composition according to claim 9 further comprising a pharmaceutically acceptable carrier and/or excipient.

11. A biosensor comprising the aptamer according to any of claims 1 to 6.

12. The aptamer according to any one of claims 1 to 6, or the genetic construct according to claim 7 or the vector according to claim 8 or the composition according to any of claims 9 or 10 for use as a medicament.

13. The aptamer according to any of claims 1 to 6, or the genetic construct according to claim 7 or the vector according to claim 8 or the composition according to any of claims 9 or 10 for use in the treatment of hepatitis C.

14. Use of the aptamer according to any of claims 1 to 6, or the genetic construct according to claim 7 or the vector according to claim 8 or the biosensor according to claim 11 for the *in vitro* diagnosis of hepatitis C.

15. Method for the *in vitro* detection or diagnosis of hepatitis C infection wherein the method comprises:
(i) contacting an isolated biological sample from a subject with the aptamer according to any one of claims 1 to 6 or the biosensor according to claim 11, and
(ii) detecting the binding of the aptamer to the core protein of the hepatitis C virus,
wherein the binding between the aptamer and the core protein of the hepatitis C virus present in the biological sample is indicative of hepatitis C infection.
